# EUROPEAN PATENT SPECIFICATION

(11) **EP 4 234 526 B1**
(45) Date of publication and mention of the grant of the patent: **25.06.2025**
(21) Application number: 21902732.3
(22) Date of filing: 10.12.2021
(51) Int. Cl.: C07C 29/74, C07C 31/08, C07D 309/40, B01D 3/14, B01D 3/40, B01J 19/24

(54) **DEVICE FOR ALCOHOL DISTILLATION DECOLORING AND PURIFICATION AND METHOD FOR PURIFICATION IN MALTOL PRODUCTION**
VORRICHTUNG ZUR ENTFÄRBUNG UND REINIGUNG DURCH ALKOHOLDESTILLATION UND VERFAHREN ZUR REINIGUNG BEI DER MALTOLHERSTELLUNG
DISPOSITIF DE DÉCOLORATION ET DE PURIFICATION PAR DISTILLATION D'ALCOOL ET PROCÉDÉ DE PURIFICATION DANS LA PRODUCTION DE MALTOL

(30) Priority: 11.12.2020 CN 202011451866
(43) Date of publication of application: 30.08.2023
(73) Proprietor: Anhui Jinhe Industrial Co., Ltd., Chuzhou, Anhui 239200 (CN)
(72) Inventor: CHEN, Yuean, Chuzhou, Anhui 239200 (CN); ZHAN, Jian, Chuzhou, Anhui 239200 (CN); ZHANG, Qijun, Chuzhou, Anhui 239200 (CN)
(74) Representative: Ipside
(86) International application number: PCT/CN2021/137214
(87) International publication number: WO 2022/122027

(56) References cited:
- CN-A- 101 747 146
- CN-A- 106 986 745
- CN-A- 112 409 133
- CN-U- 202 605 762
- CN-U- 205 182 700
- JP-A- S52 122 312
- RU-C1- 2 081 650
- "Comprehensive Utilization of Cane Sugar Factory", 31 October 1998, ISBN: 7-5019-2208-X, article LI, XILIU : "Theoretical Basis of Distillation and Impurity-Removal of Alcohol", pages: 29 - 34, XP009538152
- ZHU HUIYUN, HUANG BAOLI: "Improvement of Several Solvent Recovery Processes in Maltol Production", AN HUI HUA GONG = ANHUI CHEMICAL INDUSTRY, CN, 31 December 2001 (2001-12-31), CN , pages 22 - 23, XP055940852, ISSN: 1008-553X

## Description

This application claims priority to a Chinese patent application with an application date of December 11, 2020 and application number of 202011451866.6, entitled as device for distillation decolorization and purification of alcohol and method for purification in maltol production.

### Technical Field

The present invention relates to a device for distillation decolorization and purification of alcohol and a method for purification in maltol production.

### Background of the Invention

The production process of maltol is divided into Grignard section, chlorination section, sublimation section, crystallization section, and drying section. In the crystallization section, alcohol is mainly used to recrystallize the material to remove impurities and odors in the material so as to improve product quality. After replacement, the alcohol is recovered by being distilled in a distillation kettle. But, the quality of the distilled alcohol is not good, which affects the quality of the product, and increase in replacement efforts will cause the cost to rise.

Chinese patent document CN103570497B discloses an inorganic membrane recovering alcohol treatment method, comprising the following steps: (a) by preliminary distilling, obtaining the acidic alcohol to be treated; (b) after pumping acidic alcohol into a second reactor, adding alkali to neutralize it until the pH value reaches 9 to obtain neutral alcohol; (c) pumping the neutral alcohol into an evaporator to extract neutral gaseous hydrous alcohol; (d) dehydrating the gaseous hydrous alcohol through an inorganic membrane dehydration device, extracting dehydrated ethanol with moisture content below 0.01% to an alcohol product receiving tank. Chinese patent document CN205182700U discloses an alcohol recovery and reuse system, wherein one-time recovery of ethanol solvent used in process of alkalization, acidification, refining purification and drying is carried out by rectification column equipment to recovery simultaneously ethanol in both gaseous and liquid forms. However, the above technology still has the problem of high cost during use.

### Summary of the Invention

The first technical problem to be solved by this invention is to provide a device for distillation decolorization and purification of alcohol in maltol production, which is simple in structure, easy to use and has low cost in use.

The second technical problem to be solved by this invention is to provide a purification method using above device for distillation decolorization and purification of alcohol in maltol production to obtain alcohol with high purity and less impurity.

For solving above-mentioned first technical problem, the invention provides a device for distillation decolorization and purification of alcohol in maltol production comprising a first feed pump, a rectification column, a first condenser, a reflux tank, a first reflux pump, a first reboiler, a first extraction pump, an alkali hydrolysis kettle, a second condenser, an alkali hydrolysis receiving tank, a second feed pump, an alkali hydrolysis rectification column, a third condenser, a finished product tank, a second reflux pump, a second reboiler, and a second extraction pump. The first feed pump is connected to an inlet of the rectification column, the first condenser is connected to an upper outlet of the rectification column, the other end of the first condenser is connected to an inlet of the reflux tank, an outlet of the reflux tank is connected to the rectification column through the first reflux pump, a lower part of the rectification column is connected to the first reboiler through a circulation pipeline, bottom of the rectification column is connected to the first production pump, an outlet of the reflux tank is connected to an inlet of the alkali hydrolysis kettle through the other pipeline, the alkali hydrolysis kettle is connected to the second condenser through a circulation pipeline, an outlet of the alkali hydrolysis kettle is connected to an inlet of the alkali hydrolysis receiving tank, an outlet of the alkali hydrolysis receiving tank is connected to an inlet of the alkali hydrolysis rectification column through the second feed pump, an upper outlet of the alkali hydrolysis rectification column is connected to the third condenser, the other end of the third condenser is connected to an inlet of the finished product tank, an outlet of the finished product tank is connected to the alkali hydrolysis rectification column through the second reflux pump, a lower part of the alkali hydrolysis rectification column is connected to the second reboiler through a circulation pipeline, and bottom of the alkali hydrolysis rectification column is connected to the second extraction pump.

For the sake of simplicity, device for distillation decolorization and purification of alcohol in maltol production described hereinafter is referred to as the present device.

The present device has a simple structure, is easy to use and has low cost of use.

For solving above-mentioned second technical problem, the invention provides a purification method using the above device for distillation decolorization and purification of alcohol in maltol production, comprising the following steps:
(1) pumping crude alcohol into the rectification column at a speed of 1.5 m³/h by the first feed pump, heating the crude alcohol in the rectification column by the first reboiler with temperature of 85-87°C at the bottom of the rectification column and temperature of 76-78°C at the top of the rectification column, condensing the material distilled from the rectification column by the first condenser and sending the material into the reflux tank, refluxing one part of the material in the reflux tank and extracting the other part of the material into the alkali hydrolysis kettle with a reflux ratio of 0.6, and extracting the waste at the bottom of the rectification column by the first extraction pump;
(2) adding caustic soda into the alkali hydrolysis kettle with amount of 25kg caustic soda per 4m³ material, increasing temperature up to 77-78°C, holding the temperature for 1h to finish alkali hydrolysis, and sending the material into the alkali hydrolysis receiving tank;
(3) pumping the material in the alkali hydrolysis receiving tank into the alkali hydrolysis rectification column at a speed of 1 m³/h by the second feed pump, heating the material in the alkali hydrolysis rectification column by a second reboiler with temperature of 80-82°C at the bottom of the alkali hydrolysis rectification column and temperature of 77-78°C at the top of the alkali hydrolysis rectification column, condensing the material distilled from the rectification column by the second condenser and sending the material into the finished product tank, refluxing one part of the material in the finished product tank with a reflux ratio of 1.5, extracting qualified alcohol in the finished product tank after sampled and tested, and extracting the waste at the bottom of the alkali hydrolysis rectification column by the second extraction pump.

For the sake of simplicity, the purification method using the present device for distillation decolorization and purification of alcohol in maltol production described hereinafter is referred to as the present method.

The advantage of the present method: 1. the alcohol content after processing by the present method can reach more than 95%, and impurity content is low simultaneously; 2. the alcohol obtained by distilling has good transparency, has no peculiar smell, and is recovered to crystallization, which improves the odor quality of the finished maltol; 3. the alcohol after rectification can be sold out, which reduces the cost compared with the previous discharge into wastewater.

### Brief description of the Drawings

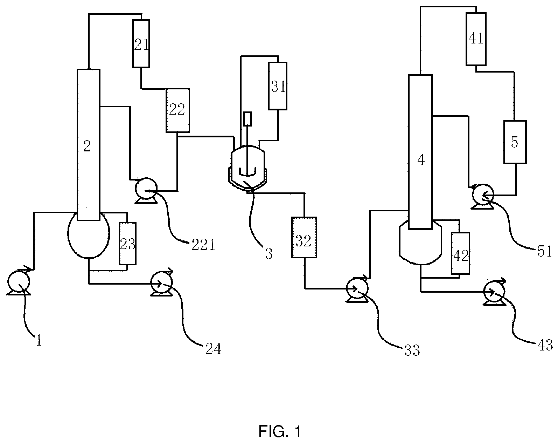

FIG. 1 is a schematic diagram of the process of the invention.

### Detailed Description of Embodiments

### Embodiment one:

Referring to Fig. 1, a device for distillation decolorization and purification of alcohol in maltol production comprises a first feed pump 1, a rectification column 2, a first condenser 21, a reflux tank 22, a first reflux pump 221, a first reboiler 23, a first extraction pump 24, an alkali hydrolysis kettle 3, a second condenser 31, an alkali hydrolysis receiving tank 32, a second feed pump 33, an alkali hydrolysis rectification column 4, a third condenser 41, a finished product tank 5, a second reflux pump 51, a second reboiler 42, and a second extraction pump 43. The first feed pump 1 is connected to an inlet of the rectification column 2, the first condenser 21 is connected to an upper outlet of the rectification column 2, the other end of the first condenser 21 is connected to an inlet of the reflux tank 22, an outlet of the reflux tank 22 is connected to the rectification column 2 through the first reflux pump 221, a lower part of the rectification column 2 is connected to the first reboiler 23 through a circulation pipeline, bottom of the rectification column 2 is connected to the first production pump 24, an outlet of the reflux tank 22 is connected to an inlet of the alkali hydrolysis kettle 3 through the other pipeline, the alkali hydrolysis kettle 3 is connected to the second condenser 31 through a circulation pipeline, an outlet of the alkali hydrolysis kettle 3 is connected to an inlet of the alkali hydrolysis receiving tank 32, an outlet of the alkali hydrolysis receiving tank 32 is connected to an inlet of the alkali hydrolysis rectification column 4 through the second feed pump 33, an upper outlet of the alkali hydrolysis rectification column 4 is connected to the third condenser 41, the other end of the third condenser 41 is connected to an inlet of the finished product tank 5, an outlet of the finished product tank 5 is connected to the alkali hydrolysis rectification column 4 through the second reflux pump 51, a lower part of the alkali hydrolysis rectification column 4 is connected to the second reboiler 42 through a circulation pipeline, and bottom of the alkali hydrolysis rectification column 4 is connected to the second extraction pump 43.

### Embodiment two:

A purification method using the device for distillation decolorization and purification of alcohol in maltol production comprises the following steps:
(1) pumping crude alcohol into the rectification column at a speed of 1.5 m³/h by the first feed pump, heating the crude alcohol in the rectification column by the first reboiler with temperature of 85°C at the bottom of the rectification column and temperature of 76°C at the top of the rectification column, condensing the material distilled from the rectification column by the first condenser and sending the material into the reflux tank, refluxing one part of the material in the reflux tank and extracting the other part of the material into the alkali hydrolysis kettle with a reflux ratio of 0.6, and extracting the waste at the bottom of the rectification column by the first extraction pump;
(2) adding caustic soda into the alkali hydrolysis kettle with amount of 25kg caustic soda per 4m³ material, increasing temperature up to 77°C, holding the temperature for 1h to finish alkali hydrolysis, and sending the material into the alkali hydrolysis receiving tank;
(3) pumping the material in the alkali hydrolysis receiving tank into the alkali hydrolysis rectification column at a speed of 1 m³/h by the second feed pump, heating the material in the alkali hydrolysis rectification column by a second reboiler with temperature of 80°C at the bottom of the alkali hydrolysis rectification column and temperature of 77°C at the top of the alkali hydrolysis rectification column, condensing the material distilled from the rectification column by the second condenser and sending the material into the finished product tank, refluxing one part of the material in the finished product tank with a reflux ratio of 1.5, extracting qualified alcohol in the finished product tank after sampled and tested, and extracting the waste at the bottom of the alkali hydrolysis rectification column by the second extraction pump.

After testing, the water content of the obtained ethanol is 0.6% and the impurity content is 3.2%.

### Embodiment three:

A purification method using the device for distillation decolorization and purification of alcohol in maltol production comprises the following steps:
(1) pumping crude alcohol into the rectification column at a speed of 1.5 m³/h by the first feed pump, heating the crude alcohol in the rectification column by the first reboiler with temperature of 86°C at the bottom of the rectification column and temperature of 76°C at the top of the rectification column, condensing the material distilled from the rectification column by the first condenser and sending the material into the reflux tank, refluxing one part of the material in the reflux tank and extracting the other part of the material into the alkali hydrolysis kettle with a reflux ratio of 0.6, and extracting the waste at the bottom of the rectification column by the first extraction pump;
(2) adding caustic soda into the alkali hydrolysis kettle with amount of 25kg caustic soda per 4m³ material, increasing temperature up to 77.5°C, holding the temperature for 1h to finish alkali hydrolysis, and sending the material into the alkali hydrolysis receiving tank;
(3) pumping the material in the alkali hydrolysis receiving tank into the alkali hydrolysis rectification column at a speed of 1 m³/h by the second feed pump, heating the material in the alkali hydrolysis rectification column by a second reboiler with temperature of 81°C at the bottom of the alkali hydrolysis rectification column and temperature of 77.5°C at the top of the alkali hydrolysis rectification column, condensing the material distilled from the rectification column by the second condenser and sending the material into the finished product tank, refluxing one part of the material in the finished product tank with a reflux ratio of 1.5, extracting qualified alcohol in the finished product tank after sampled and tested, and extracting the waste at the bottom of the alkali hydrolysis rectification column by the second extraction pump.

After testing, the water content of the obtained ethanol is 0.8% and the impurity content is 3.1%.

### Embodiment four:

A purification method using the device for distillation decolorization and purification of alcohol in maltol production comprises the following steps:
(1) pumping crude alcohol into the rectification column at a speed of 1.5 m³/h by the first feed pump, heating the crude alcohol in the rectification column by the first reboiler with temperature of 87°C at the bottom of the rectification column and temperature of 78°C at the top of the rectification column, condensing the material distilled from the rectification column by the first condenser and sending the material into the reflux tank, refluxing one part of the material in the reflux tank and extracting the other part of the material into the alkali hydrolysis kettle with a reflux ratio of 0.6, and extracting the waste at the bottom of the rectification column by the first extraction pump;
(2) adding caustic soda into the alkali hydrolysis kettle with amount of 25kg caustic soda per 4m³ material, increasing temperature up to 78°C, holding the temperature for 1h to finish alkali hydrolysis, and sending the material into the alkali hydrolysis receiving tank;
(3) pumping the material in the alkali hydrolysis receiving tank into the alkali hydrolysis rectification column at a speed of 1 m³/h by the second feed pump, heating the material in the alkali hydrolysis rectification column by a second reboiler with temperature of 82°C at the bottom of the alkali hydrolysis rectification column and temperature of 78°C at the top of the alkali hydrolysis rectification column, condensing the material distilled from the rectification column by the second condenser and sending the material into the finished product tank, refluxing one part of the material in the finished product tank with a reflux ratio of 1.5, extracting qualified alcohol in the finished product tank after sampled and tested, and extracting the waste at the bottom of the alkali hydrolysis rectification column by the second extraction pump.

After testing, the water content of the obtained ethanol is 1.0% and the impurity content is 3.0%.

The above described are only specific embodiments of the present application, under the above-mentioned teachings of the application, those skilled in the art can carry out other improvements or deformations on the basis of the above-mentioned embodiments. Those skilled in the art should understand that the above specific description is only to better explain the purpose of the present application, and the protection scope of the present application should be subject to the protection scope of the claims.

Furthermore, those skilled in the art can understand that although some embodiments described herein include some features included in other embodiments but not others, the combination of features of different embodiments means that they are within the scope of the present application and form different embodiments. The present invention is only limited by the scope of the appended claims.

## Claims

1. A device for distillation decolorization and purification of alcohol in maltol production, is **characterized in that**, comprising a rectification column, an alkali hydrolysis kettle, and an alkali hydrolysis rectification column; an upper outlet of the rectification column is connected to an inlet of the alkali hydrolysis kettle, an outlet of the alkali hydrolysis kettle is connected to an inlet of the alkali hydrolysis rectification column, and an upper outlet of the alkali hydrolysis rectification column is connected to an inlet of a finished product tank.

2. The device for distillation decolorization and purification of alcohol in maltol production according to claim 1, is **characterized in that**, further comprising a first condenser, a second condenser, and a third condenser, the first condenser is connected to an upper outlet of the rectification column, the alkali hydrolysis tank is connected to the second condenser through a circulation pipeline, and an upper outlet of the alkali hydrolysis rectification column is connected to the third condenser.

3. The device for distillation decolorization and purification of alcohol in maltol production according to claim 2, is **characterized in that**, the other end of the first condenser other end is connected to an inlet of the reflux tank, an outlet of the reflux tank is connected to the rectification column through the first reflux pump, and a lower part of the rectification column is connected to the first reboiler through a circulation pipeline.

4. The device for distillation decolorization and purification of alcohol in maltol production according to claim 2, is **characterized in that**, the other end of the third condenser is connected an inlet of the finished product tank, an outlet of the finished product tank is connected to the alkali hydrolysis rectification column through the second reflux pump, a lower part of the alkali hydrolysis rectification column is connected to the second reboiler through a circulation pipeline, and bottom of the alkali hydrolysis rectification column is connected to the second extraction pump.

5. The device for distillation decolorization and purification of alcohol in maltol production according to claim 1, is **characterized in that**, further comprising a first feed pump, a first condenser, a reflux tank, a first reflux pump, a first reboiler, a first extraction pump, a second condenser, an alkali hydrolysis receiving tank, a second feed pump, a third condenser, a finished product tank, a second reflux pump, a second reboiler, and a second extraction pump, the first feed pump is connected to an inlet of the rectification column, the first condenser is connected to an upper outlet of the rectification column, the other end of the first condenser is connected to an inlet of the reflux tank, an outlet of the reflux tank is connected to the rectification column through the first reflux pump, a lower part of the rectification column is connected to the first reboiler through a circulation pipeline, bottom of the rectification column is connected to the first production pump, an outlet of the reflux tank is connected to an inlet of the alkali hydrolysis kettle through the other pipeline, the alkali hydrolysis kettle is connected to the second condenser through a circulation pipeline, an outlet of the alkali hydrolysis kettle is connected to an inlet of the alkali hydrolysis receiving tank, an outlet of the alkali hydrolysis receiving tank is connected to an inlet of the alkali hydrolysis rectification column through the second feed pump, an upper outlet of the alkali hydrolysis rectification column is connected to the third condenser, the other end of the third condenser is connected to an inlet of the finished product tank, an outlet of the finished product tank is connected to the alkali hydrolysis rectification column through the second reflux pump, a lower part of the alkali hydrolysis rectification column is connected to the second reboiler through a circulation pipeline, and bottom of the alkali hydrolysis rectification column is connected to the second extraction pump.

6. A purification method using the device for distillation decolorization and purification of alcohol in maltol production according to any one of claims 1 to 5, is **characterized in that**, comprising the following steps:
(1) pumping crude alcohol into the rectification column at a speed of 1.5 m³/h by the first feed pump, heating the crude alcohol in the rectification column by the first reboiler with temperature of 85-87°C at the bottom of the rectification column and temperature of 76-78°C at the top of the rectification column, condensing the material distilled from the rectification column by the first condenser and sending the material into the reflux tank, refluxing one part of the material in the reflux tank and extracting the other part of the material into the alkali hydrolysis kettle with a reflux ratio of 0.6, and extracting the waste at the bottom of the rectification column by the first extraction pump;
(2) adding caustic soda into the alkali hydrolysis kettle with amount of 25kg caustic soda per 4m³ material, increasing temperature up to 77-78°C, holding the temperature for 1h to finish alkali hydrolysis, and sending the material into the alkali hydrolysis receiving tank;
(3) pumping the material in the alkali hydrolysis receiving tank into the alkali hydrolysis rectification column at a speed of 1 m³/h by the second feed pump, heating the material in the alkali hydrolysis rectification column by a second reboiler with temperature of 80-82°C at the bottom of the alkali hydrolysis rectification column and temperature of 77-78°C at the top of the alkali hydrolysis rectification column, condensing the material distilled from the rectification column by the second condenser and sending the material into the finished product tank, refluxing one part of the material in the finished product tank with a reflux ratio of 1.5, extracting qualified alcohol in the finished product tank after sampled and tested, and extracting the waste at the bottom of the alkali hydrolysis rectification column by the second extraction pump.

7. A purification method using the device for distillation decolorization and purification of alcohol in maltol production according to any of claims 1 to 5 is **characterized in that**, comprising the following steps:
Step 1: pumping crude alcohol into the rectification column with temperature of 85-87°C at the bottom of the rectification column and temperature of 76-78°C at the top of the rectification column, refluxing one part of the material distilled from the rectification column, extracting the other part of the material into the alkali hydrolysis kettle with a reflux ratio of 0.6;
Step 2: adding caustic soda into the alkali hydrolysis kettle, increasing temperature up to 77-78°C, and holding the temperature to finish alkali hydrolysis;
Step 3: sending the material processed by holding to finish alkali hydrolysis in step 2 to the alkali hydrolysis rectification column with temperature of 80-82°C at the bottom of the alkali hydrolysis rectification column and temperature of 77-78°C at the top of the alkali hydrolysis rectification column, refluxing one part of the material distilled from the alkali hydrolysis rectification column with a reflux ratio of 1.5.

8. A purification method using the device for distillation decolorization and purification of alcohol in maltol production according to claim 7, is **characterized in that**, comprising the following steps: the reflux ratio in step 1 is 0.6.

9. A purification method using the device for distillation decolorization and purification of alcohol in maltol production according to claim 7, is **characterized in that**, comprising the following steps: the reflux ratio in step 3 is 1.5.

## Patentansprüche

1. Vorrichtung zur destillativen Entfärbung und Reinigung von Alkohol bei der Maltolherstellung, **dadurch gekennzeichnet, dass** sie eine Rektifikationskolonne, einen Alkali-Hydrolysekessel und eine Alkali-Hydrolyse-Rektifikationskolonne umfasst; ein oberer Auslass der Rektifikationskolonne mit einem Einlass des Alkali-Hydrolysekessels verbunden ist, ein Auslass des Alkali-Hydrolysekessels mit einem Einlass der Alkali-Hydrolyse-Rektifikationskolonne verbunden ist und ein oberer Auslass der Alkali-Hydrolyse-Rektifikationskolonne mit einem Einlass eines Fertigprodukttanks verbunden ist.

2. Vorrichtung zur destillativen Entfärbung und Reinigung von Alkohol bei der Maltolherstellung nach Anspruch 1, **dadurch gekennzeichnet, dass** sie weiter einen ersten Kondensator, einen zweiten Kondensator und einen dritten Kondensator umfasst, der erste Kondensator mit einem oberen Auslass der Rektifikationskolonne verbunden ist, der Alkali-Hydrolysetank über eine Umlaufleitung mit dem zweiten Kondensator verbunden ist und ein oberer Auslass der Alkali-Hydrolyse-Rektifikationskolonne mit dem dritten Kondensator verbunden ist.

3. Vorrichtung zur destillativen Entfärbung und Reinigung von Alkohol bei der Maltolherstellung nach Anspruch 2, **dadurch gekennzeichnet, dass** das andere Ende des ersten anderen Endes des Kondensators mit einem Einlass des Rückflusstanks verbunden ist, ein Auslass des Rückflusstanks über die erste Rückflusspumpe mit der Rektifikationskolonne verbunden ist und ein unterer Teil der Rektifikationskolonne über eine Umlaufleitung mit dem ersten Reboiler verbunden ist.

4. Vorrichtung zur destillativen Entfärbung und Reinigung von Alkohol bei der Maltolherstellung nach Anspruch 2, **dadurch gekennzeichnet, dass** das andere Ende des dritten Kondensators mit einem Einlass des Fertigprodukttanks verbunden ist, ein Auslass des Fertigprodukttanks über die zweite Rückflusspumpe mit der Alkali-Hydrolyse-Rektifikationskolonne verbunden ist, ein unterer Teil der Alkali-Hydrolyse-Rektifikationskolonne über eine Umlaufleitung mit dem zweiten Reboiler verbunden ist, und die Unterseite der Alkali-Hydrolyse-Rektifikationskolonne mit der zweiten Extraktionspumpe verbunden ist.

5. Vorrichtung zur destillativen Entfärbung und Reinigung von Alkohol bei der Maltolherstellung nach Anspruch 1, **dadurch gekennzeichnet, dass** sie weiter eine erste Speisepumpe, einen ersten Kondensator, einen Rückflusstank, eine erste Rückflusspumpe, einen ersten Reboiler, eine erste Extraktionspumpe, einen zweiten Kondensator, einen Alkali-Hydrolyse-Aufnahmetank, eine zweite Speisepumpe, einen dritten Kondensator, einen Fertigprodukttank, eine zweite Rückflusspumpe, einen zweiten Reboiler und eine zweite Extraktionspumpe umfasst, die erste Speisepumpe mit einem Einlass der Rektifikationskolonne verbunden ist, der erste Kondensator mit einem oberen Auslass der Rektifikationskolonne verbunden ist, das andere Ende des ersten Kondensators mit einem Einlass des Rückflusstanks verbunden ist, ein Auslass des Rückflusstanks über die erste Rückflusspumpe mit der Rektifikationskolonne verbunden ist, ein unterer Teil der Rektifikationskolonne über eine Umlaufleitung mit dem ersten Reboiler verbunden ist, die Unterseite der Rektifikationskolonne mit der ersten Produktionspumpe verbunden ist, ein Auslass des Rückflusstanks über die andere Leitung mit einem Einlass des Alkali-Hydrolysekessels verbunden ist, der Alkali-Hydrolysekessel über eine Umlaufleitung mit dem zweiten Kondensator verbunden ist, ein Auslass des Alkali-Hydrolysekessels mit einem Einlass des Alkali-Hydrolyse-Aufnahmetanks verbunden ist, ein Auslass des Alkali-Hydrolyse-Aufnahmetanks über die zweite Speisepumpe mit einem Einlass der Alkali-Hydrolyse-Rektifikationskolonne verbunden ist, ein oberer Auslass der Alkali-Hydrolyse-Rektifikationskolonne mit dem dritten Kondensator verbunden ist, das andere Ende des dritten Kondensators mit einem Einlass des Fertigprodukttanks verbunden ist, ein Auslass des Fertigprodukttanks über die zweite Rückflusspumpe mit der Alkali-Hydrolyse-Rektifikationskolonne verbunden ist, ein unterer Teil der Alkali-Hydrolyse-Rektifikationskolonne über eine Umlaufleitung mit dem zweiten Reboiler verbunden ist, und die Unterseite der Alkali-Hydrolyse-Rektifikationskolonne mit der zweiten Extraktionspumpe verbunden ist.

6. Reinigungsverfahren unter Verwendung der Vorrichtung zur destillativen Entfärbung und Reinigung von Alkohol bei der Maltolherstellung nach einem der Ansprüche 1 bis 5, **dadurch gekennzeichnet, dass** es die folgenden Schritte umfasst:
(1) Pumpen von Rohalkohol in die Rektifikationskolonne mit einer Geschwindigkeit von 1,5 m³/h durch die erste Speisepumpe, Erhitzen des Rohalkohols in der Rektifikationskolonne durch den ersten Reboiler mit einer Temperatur von 85-87 °C an der Unterseite der Rektifikationskolonne und einer Temperatur von 76-78 °C an der Oberseite der Rektifikationskolonne, Kondensieren des aus der Rektifikationskolonne destillierten Materials durch den ersten Kondensator und Senden des Materials in den Rückflusstank, Zurückfließen eines Teils des Materials in den Rückflusstank und Extrahieren des anderen Teils des Materials in den Alkali-Hydrolysekessel mit einem Rückflussverhältnis von 0,6, und Extrahieren des Abfalls an der Unterseite der Rektifikationskolonne durch die erste Extraktionspumpe;
(2) Zugeben von Natronlauge in den Alkali-Hydrolysekessel mit einer Menge von 25 kg Natronlauge pro 4 m³ Material, Erhöhen der Temperatur auf 77-78 °C, Halten der Temperatur über 1h, um die Alkali-Hydrolyse abzuschließen, und Senden des Materials in den Alkali-Hydrolyse-Aufnahmetank;
(3) Pumpen des Materials im Alkali-Hydrolyse-Aufnahmetank in die Alkali-Hydrolyse-Rektifikationskolonne mit einer Geschwindigkeit von 1 m³/h durch die zweite Speisepumpe, Erhitzen des Materials in der Alkali-Hydrolyse-Rektifikationskolonne durch einen zweiten Reboiler mit einer Temperatur von 80-82 °C an der Unterseite der Alkali-Hydrolyse-Rektifikationskolonne und einer Temperatur von 77-78 °C an der Oberseite der Alkali-Hydrolyse-Rektifikationskolonne, Kondensieren des aus der Rektifikationskolonne destillierten Materials durch den zweiten Kondensator und Senden des Materials in den Fertigprodukttank, Zurückfließen eines Teils des Materials in den Fertigprodukttank mit einem Rückflussverhältnis von 1,5, Extrahieren von qualifiziertem Alkohol im Fertigprodukttank nach Probenahme und Prüfung, und Extrahieren des Abfalls an der Unterseite der Alkali-Hydrolyse-Rektifikationskolonne durch die zweite Extraktionspumpe.

7. Reinigungsverfahren unter Verwendung der Vorrichtung zur destillativen Entfärbung und Reinigung von Alkohol bei der Maltolherstellung nach einem der Ansprüche 1 bis 5, **dadurch gekennzeichnet, dass** es die folgenden Schritte umfasst:
Schritt 1: Pumpen von Rohalkohol in die Rektifikationskolonne mit einer Temperatur von 85-87 °C an der Unterseite der Rektifikationskolonne und einer Temperatur von 76-78 °C an der Oberseite der Rektifikationskolonne, Zurückfließen eines Teils des aus der Rektifikationskolonne destillierten Materials, Extrahieren des anderen Teils des Materials in den Alkali-Hydrolysekessel mit einem Rückflussverhältnis von 0,6;
Schritt 2: Zugeben von Natronlauge in den Alkali-Hydrolysekessel, Erhöhen der Temperatur auf 77-78 °C und Halten der Temperatur, um die Alkali-Hydrolyse abzuschließen;
Schritt 3: Senden des Materials, das durch Halten verarbeitet wurde, um die Alkalihydrolyse in Schritt 2 abzuschließen, in die Alkali-Hydrolyse-Rektifikationskolonne mit einer Temperatur von 80-82 °C an der Unterseite der Alkali-Hydrolyse-Rektifikationskolonne und einer Temperatur von 77-78 °C an der Oberseite der Alkali-Hydrolyse-Rektifikationskolonne, Zurückfließen eines Teils des aus der Alkali-Hydrolyse-Rektifikationskolonne destillierten Materials mit einem Rückflussverhältnis von 1,5.

8. Reinigungsverfahren unter Verwendung der Vorrichtung zur destillativen Entfärbung und Reinigung von Alkohol bei der Maltolherstellung nach Anspruch 7, **dadurch gekennzeichnet, dass** es die folgenden Schritte umfasst: das Rückflussverhältnis in Schritt 1 beträgt 0,6.

9. Reinigungsverfahren unter Verwendung der Vorrichtung zur destillativen Entfärbung und Reinigung von Alkohol bei der Maltolherstellung nach Anspruch 7, **dadurch gekennzeichnet, dass** es die folgenden Schritte umfasst: das Rückflussverhältnis in Schritt 3 beträgt 1,5.

## Revendications

1. Dispositif de décoloration et de purification par distillation d'alcool dans la production de maltol, **caractérisé en ce qu'**il comporte une colonne de rectification, une cuve d'hydrolyse alcaline et une colonne de rectification d'hydrolyse alcaline ; une sortie supérieure de la colonne de rectification est reliée à une entrée de la cuve d'hydrolyse alcaline, une sortie de la cuve d'hydrolyse alcaline est reliée à une entrée de la colonne de rectification d'hydrolyse alcaline, et une sortie supérieure de la colonne de rectification d'hydrolyse alcaline est reliée à une entrée d'un réservoir de produit fini.

2. Dispositif de décoloration et de purification par distillation d'alcool dans la production de maltol selon la revendication 1, **caractérisé en ce qu'**il comporte en outre un premier condenseur, un deuxième condenseur et un troisième condenseur, le troisième condenseur est relié à une sortie supérieure de la colonne de rectification, la cuve d'hydrolyse alcaline est reliée au deuxième condenseur par le biais d'une canalisation de circulation, et une sortie supérieure de la colonne de rectification d'hydrolyse alcaline est reliée au troisième condenseur.

3. Dispositif de décoloration et de purification par distillation d'alcool dans la production de maltol selon la revendication 2, **caractérisé en ce que** l'autre extrémité de l'autre extrémité du premier condenseur est reliée à une entrée du réservoir de reflux, une sortie du réservoir de reflux est reliée à la colonne de rectification par le biais de la première pompe de reflux, et une partie inférieure de la colonne de rectification est reliée au premier rebouilleur par le biais d'une canalisation de circulation.

4. Dispositif de décoloration et de purification par distillation d'alcool dans la production de maltol selon la revendication 2, **caractérisé en ce que** l'autre extrémité du troisième condenseur est reliée à une entrée du réservoir de produit fini, une sortie du réservoir de produit fini est reliée à la colonne de rectification d'hydrolyse alcaline par le biais de la deuxième pompe de reflux, une partie inférieure de la colonne de rectification d'hydrolyse alcaline est reliée au deuxième rebouilleur par le biais d'une canalisation de circulation, et un fond de la colonne de recirculation d'hydrolyse alcaline est reliée à la deuxième pompe d'extraction.

5. Dispositif de décoloration et de purification par distillation d'alcool dans la production de maltol selon la revendication 1, **caractérisé en ce qu'**il comporte en outre une première pompe d'alimentation, un premier condenseur, un réservoir de reflux, une première pompe de reflux, un premier rebouilleur, une première pompe d'extraction, un deuxième condenseur, un réservoir de réception d'hydrolyse alcaline, une deuxième pompe d'alimentation, un troisième condenseur, un réservoir de produit fini, une deuxième pompe de reflux, un deuxième rebouilleur et une deuxième pompe d'extraction, la première pompe d'alimentation est reliée à une entrée de la colonne de rectification, le premier condenseur est relié à une sortie supérieure de la colonne de rectification, l'autre extrémité du premier condenseur est reliée à une entrée du réservoir de reflux, une sortie du réservoir de reflux est reliée à la colonne de rectification par le biais de la première pompe de reflux, une partie inférieure de la colonne de rectification et reliée au premier rebouilleur par le biais d'une canalisation de circulation, un fond de la colonne de rectification est relié à la première pompe de production, une sortie du réservoir de reflux est reliée à une entrée de la cuve d'hydrolyse alcaline par le biais de l'autre canalisation, la cuve d'hydrolyse alcaline est reliée au deuxième condenseur par le biais d'une canalisation de circulation, une sortie de la cuve d'hydrolyse alcaline est reliée à une entrée du réservoir de réception d'hydrolyse alcaline, une sortie du réservoir de réception d'hydrolyse alcaline est reliée à une entrée de la colonne de rectification d'hydrolyse alcaline par le biais de la deuxième pompe d'alimentation, une sortie supérieure de la colonne de rectification d'hydrolyse alcaline est reliée au troisième condenseur, l'autre extrémité du troisième condenseur est reliée à une entrée du réservoir de produit fini, une sortie du réservoir de produit fini est reliée à la colonne de rectification d'hydrolyse alcaline par le biais de la deuxième pompe de reflux, une partie inférieure de la colonne de rectification d'hydrolyse alcaline est reliée au deuxième rebouilleur par le biais d'une canalisation de circulation, et un fond de la colonne de rectification d'hydrolyse alcaline est relié à la deuxième pompe d'extraction.

6. Procédé de purification utilisant le dispositif de décoloration et de purification par distillation d'alcool dans la production de maltol selon l'une quelconque des revendications 1 à 5, **caractérisé en ce qu'**il comporte les étapes suivantes :
(1) le pompage d'alcool brut pour l'injecter dans la colonne de rectification à une vitesse de 1,5 m³/h par la première pompe d'alimentation, le chauffage de l'alcool brut dans la colonne de rectification par le premier rebouilleur à une température de 85-87 °C au fond de la colonne de rectification et à une température de 76-78 °C au sommet de la colonne de rectification, la condensation du matériau distillé provenant de la colonne de rectification par le premier condenseur et l'envoi du matériau dans le réservoir de reflux, le reflux d'une partie du matériau contenu dans le réservoir de reflux et l'extraction de l'autre partie du matériau dans la cuve d'hydrolyse alcaline à un taux de reflux de 0,6, et l'extraction des déchets au fond de la colonne de rectification par la première pompe d'extraction ;
(2) l'ajout de soude caustique dans la cuve d'hydrolyse alcaline en une quantité de 25 kg de soude caustique pour 4 m³ de matériau, l'augmentation de la température à 77-78 °C, le maintien de la température pendant 1 h pour achever l'hydrolyse alcaline, et l'envoi du matériau dans le réservoir de réception d'hydrolyse alcaline ;
(3) le pompage du matériau contenu dans le réservoir de réception d'hydrolyse alcaline pour l'injecter dans la colonne de rectification d'hydrolyse alcaline à une vitesse de 1 m³/h par la deuxième pompe d'alimentation, le chauffage du matériau contenu dans la colonne de rectification d'hydrolyse alcaline par un deuxième rebouilleur à une température de 80-82 °C au fond de la colonne de rectification d'hydrolyse alcaline et à une température de 77-78 °C au sommet de la colonne de rectification d'hydrolyse alcaline, la condensation du matériau distillé provenant de la colonne de rectification par le deuxième condenseur et l'envoi du matériau dans le réservoir de produit fini, le reflux d'une partie du matériau contenu dans le réservoir de produit fini à un taux de reflux de 1,5, l'extraction d'alcool qualifié dans le réservoir de produit fini après l'avoir soumis à un échantillonnage et à un essai, et l'extraction des déchets au fond de la colonne de rectification d'hydrolyse alcaline par la deuxième pompe d'extraction.

7. Procédé de purification utilisant le dispositif de décoloration et de purification par distillation d'alcool dans la production de maltol selon l'une quelconque des revendications 1 à 5, **caractérisé en ce qu'**il comporte les étapes suivantes :
Étape 1 : le pompage d'alcool brut pour l'injecter dans la colonne de rectification à une température de 85-87 °C au fond de la colonne de rectification et à une température de 76-78 °C au sommet de la colonne de rectification, le reflux d'une partie du matériau distillé provenant de la colonne de rectification, l'extraction de l'autre partie du matériau dans la cuve d'hydrolyse alcaline à un taux de reflux de 0,6 ;
Étape 2 : l'ajout de soude caustique dans la cuve d'hydrolyse alcaline, l'augmentation de la température à 77-78 °C et le maintien de la température pour achever l'hydrolyse alcaline ;
Étape 3 : l'envoi du matériau traité par maintien pour achever l'hydrolyse alcaline à l'étape 2 vers la colonne de rectification d'hydrolyse alcaline à une température de 80-82 °C au fond de la colonne de rectification d'hydrolyse alcaline et à une température de 77-78 °C au sommet de la colonne de rectification d'hydrolyse alcaline, le reflux d'une partie du matériau distillé provenant de la colonne de rectification d'hydrolyse alcaline à un taux de reflux de 1,5.

8. Procédé de purification utilisant le dispositif de décoloration et de purification par distillation d'alcool dans la production de maltol selon la revendication 7, **caractérisé en ce qu'**il comporte les étapes suivantes : le taux de reflux à l'étape 1 est de 0,6.

9. Procédé de purification utilisant le dispositif de décoloration et de purification par distillation d'alcool dans la production de maltol selon la revendication 7, **caractérisé en ce qu'**il comporte les étapes suivantes : le taux de reflux à l'étape 3 est de 1,5.
